# EUROPEAN PATENT SPECIFICATION

(11) **EP 2 116 175 B1**
(45) Date of publication and mention of the grant of the patent: **23.10.2013**
(21) Application number: 09000971.3
(22) Date of filing: 23.01.2009
(51) Int. Cl.: A61B 1/018

(54) **Medical system**
Medizinisches System
Système médical

(30) Priority: 09.05.2008 US 118319
(43) Date of publication of application: 11.11.2009
(73) Proprietor: Olympus Medical Systems Corp., Tokyo 151-0072 (JP)
(72) Inventor: Shigeta, Ken, Hachioji-shi Tokyo 192-8512 (JP); Banju, Kazuo, Hachioji-shi Tokyo 192-8512 (JP)
(74) Representative: von Hellfeld, Axel

(56) References cited:
- EP-A- 1 602 319
- EP-A- 1 872 709
- EP-A1- 2 130 510
- EP-A2- 2 108 304
- WO-A-2007/138674
- US-A1- 2007 129 719
- US-A1- 2008 015 410

## Description

The present invention relates to a medical system wherein a treatment device is inserted into a body cavity through a channel of an insertion device to be inserted into the body cavity, a driving force is applied to a proximal end portion of the surgical device, the driving force is transmitted to an actuating portion at a distal end portion through a transmitting portion, and the actuating portion is moved.

A specification of U.S. Patent Application Publication No. 2005/70757 discloses a medical system. In this medical system, an endoscope is inserted into a body cavity, a forceps is inserted into the body cavity from a forceps opening of the endoscope through a forceps channel, and the forceps is used to perform a treatment. In the forceps, a grasping portion to grasp a living tissue and an insertion tube portion long and flexible are continuously provided from a distal end side to a proximal end side. When a proximal end portion of the insertion tube portion extended from the forceps opening of the endoscope is manually moved forward and backward, and rotated, the grasping portion is moved forward and backward, and rotated through the insertion tube portion inserted through the forceps channel.

In such a medical system as disclosed in the specification of U.S. Patent Application Publication No. 2005/70757 , if the proximal end portion of the insertion tube portion is not manually operated but driven by a driving device, the insertion tube portion is floating between the driving device and the forceps opening, a driving force applied to the proximal end portion of the insertion tube portion is not assuredly transmitted to the grasping portion at the distal end portion, and hence it is difficult to move the grasping portion assuredly.

WO 2007/138674 A1 discloses an endoscope treatment system having an advance/retreat device and a connection tube.

EP 1 872 709 A1 discloses an endoscope system comprising an endoscope, a treatment instrument cartridge, an advancing and retracting device, a controlling device, and an operation instructing device. EP 1 602 319 A, US 2008/015410 A1, US 2005/070757, US 2007/129719, disclose similar endoscope systems, all having advancing and retracting devices for the treatment instrument. EP 2 130 510 A1 discloses an endoscope sheath having a rotatable handle through which the endoscope is guided.

In view of the above-explained problem, it is an object of the present invention to provide a medical system wherein an actuating portion at a distal end portion of a treatment device is to be moved assuredly. Such a medical system is provided according to independent claim 1. Various improvements to the medical system are defined in the dependent claims.

In an aspect of the present invention, a medical system is provided according to claim 1. Various embodiments of the medical system are defined in the dependent claims.

In the medical system according to this aspect, since the transmitting portion is supported and guided between the coupling portion coupled with the coupling accepting portion and the proximal end opening portion by the guide member harder than the transmitting portion, the driving force applied to the coupling portion by the driving mechanism is assuredly transmitted to the actuating portion by the transmitting portion, and therefore the actuating portion is moved assuredly.

In a preferred aspect of the present invention, the medical system is **characterized in that** the coupling portion is attachable to and detachable from the coupling accepting portion, and the guide mechanism includes a retreat support mechanism supporting the guide member such that the guide member is movable to a retreat position where at least a part of the guide member retreats from a space between the coupling portion coupled with the coupling accepting portion and the proximal end opening portion.

In the medical system according to this aspect, when at least a part of the guide member retreats from the space between the coupling portion coupled with the coupling accepting portion and the proximal end opening portion, a space where the coupling portion is to be attached to and detached from the coupling accepting portion is assured, and so an attachment and detachment operation for the coupling portion with respect to the coupling accepting portion can be carried out easily.

In a preferred aspect of the present invention, the medical system is **characterized in that** the guide member includes an attachment and detachment portion through which the transmitting portion is attachable to and detachable from the guide member in a direction crossing a guiding direction along which the transmitting portion is guided by the guide member.

In the medical system according to this aspect, since the transmitting portion can be attached to and detached from the guide member through the attachment and detachment portion of the guide member in the direction crossing the guiding direction of the guide member, the attachment and detachment operation for the transmitting portion with respect to the guide member can be carried out easily.

In a preferred aspect of the present invention, the medical system is **characterized in that** the guide member includes a retreat escape portion to have at least one of the transmitting portion and the proximal end opening portion escape such that the guide member does not interfere with at least one of the proximal end opening portion and the transmitting portion extending between the coupling portion coupled with the coupling accepting portion and the proximal end opening portion in regard to the movement of the guide member to the retreat position.

In the medical system according to this aspect, the guide member does not interfere with the transmitting portion and the proximal end opening portion in the movement of the guide member.

In a preferred aspect of the present invention, the medical system is **characterized in that** the guide mechanism includes a fall-preventing member covering the retreat escape portion from an outer side of the guide member.

In the medical system according to this aspect, the fall-preventing member prevents the transmitting portion from falling off through the retreat escape portion.

In a preferred aspect of the present invention, the medical system is **characterized in that** the guide member includes a follow-up support mechanism supporting the guide member so as to be movable in a driving direction of the coupling portion and an urging mechanism urging the guide member toward a coupling accepting portion side in the driving direction of the coupling portion, and the guide member includes a contact portion provided at a coupling accepting portion side end portion, and the coupling portion includes a contact accepting portion arranged at the proximal end portion of the transmitting portion and with which the contact portion is to come into contact.

In the medical system according to this aspect, since the guide member follows the movement of the coupling portion to move in a state where the contact portion of the guide member is in contact with the contact accepting portion of the coupling portion, a space where the transmitting portion is not supported by the guide member is not formed between the coupling portion side end portion of the guide member and the coupling portion, and the transmitting portion can more assuredly transmit the driving force.

In a preferred aspect of the present invention, the medical system is **characterized in that** the guide member includes a follow-up escape portion to have at least one of the transmitting portion and the proximal end opening portion escape such that the guide member does not interfere with at least one of the proximal end opening portion and the transmitting portion extending between the coupling portion coupled with the coupling accepting portion and the proximal end opening portion in regard to the movement of the guide member in the driving direction of the coupling portion.

In the medical system according to this aspect, the guide member does not interfere with the transmitting portion and the proximal end opening portion in the movement of the guide member in the driving direction of the coupling portion.

In a preferred aspect of the present invention, the medical system is **characterized in that** the guide mechanism includes a fall-preventing member covering the follow-up escape portion from the outer side of the guide member.

In the medical system according to this aspect, the fall-preventing member prevents the transmitting portion from falling off through the follow-up escape portion.

In a preferred aspect of the present invention, the medical system is **characterized in that** the guide mechanism includes a support mechanism supporting the guide member so as to be movable in a driving direction of the coupling portion and to arrange the guide member at a retreat position where at least a part of the guide member is retreated in the driving direction of the coupling portion from a space between the coupling portion coupled with the coupling accepting portion and the proximal end opening portion, an urging mechanism urging the guide member toward a coupling accepting portion side in the driving direction of the coupling portion, and an holding mechanism to hold the guide member at the retreat position, and the guide member includes a contact portion provided at a coupling accepting portion side end portion, and the coupling portion is attachable to and detachable from the coupling accepting portion and includes a contact accepting portion arranged at the proximal end portion of the transmitting portion and with which the contact portion is to come into contact.

In the medical system according to this aspect, since the guide member follows the movement of the coupling portion to move in a state where the contact portion of the guide member is in contact with the contact accepting portion of the coupling portion, a space where the transmitting portion is not supported by the guide member is not formed between the coupling portion side end portion of the guide member and the coupling portion, and so the transmitting portion can more assuredly transmit the driving force applied to the coupling portion. Further, when at least a part of the guide member is moved from a space between the coupling accepting portion and the proximal end opening portion in the driving direction of the coupling portion and the guide member is arranged at the retreat position and held, a space where the coupling portion is to be attached to and detached from the coupling accepting portion is assured without holding the guide member against an urging force of the urging mechanism by an operator, and so the attachment and detachment operation for the coupling portion with respect to the coupling accepting portion can be carried out easily.

In a preferred aspect of the present invention, the medical system is **characterized in that** the treatment device includes an actuation mechanism provided in the actuating portion, a coupling mechanism provided in the coupling portion, and a transmission member inserted through the transmitting portion, extending between the actuation mechanism and the coupling mechanism, and to transmit a driving force applied to the coupling mechanism to the actuation mechanism to actuate the actuation mechanism, and the driving device includes a driving coupling mechanism to drive the coupling mechanism.

In the medical system according to this aspect, since the guide member harder than the transmitting portion supports and guides the transmitting portion between the coupling portion coupled with the coupling accepting portion and the proximal end opening portion, the transmission member inserted through the transmitting portion is assuredly transmit the driving force applied to the coupling driving mechanism to the actuation mechanism, and so the actuation mechanism is securely operated.

The invention can be more fully understood from the following detailed description when taken in conjunction with the accompanying drawings, in which:
FIG. 1 is a schematic view showing a medical system according to a first embodiment of the present invention;
FIG. 2 is a perspective view showing a forceps manipulator according to the first embodiment of the present invention;
FIG. 3 is a perspective view showing a driving unit according to the first embodiment of the present invention;
FIG. 4A is a longitudinal cross-sectional view showing the driving unit according to the first embodiment of the present invention;
FIG. 4B is a transverse cross-sectional view showing the driving unit according to the first embodiment of the present invention taken along a line IVB-IVB in FIG. 4A;
FIG. 5A is a side view showing the medical system in an actuation state according to the first embodiment of the present invention;
FIG. 5B is a side view showing the medical system in an attachment and detachment state according to the first embodiment of the present invention;
FIG. 6 is a longitudinal cross-sectional view showing a manipulator and a guide mechanism according to the first embodiment of the present invention;
FIG. 7 is a transverse cross-sectional view showing the manipulator and the guide mechanism according to the first embodiment of the present invention;
FIG. 8A is a side view showing a medical system in a backward movement operation state according to a second embodiment of the present invention;
FIG. 8B is a side view showing the medical system in a forward movement operation state according to the second embodiment of the present invention;
FIG. 8C is a side view showing the medical system in an attachment and detachment state according to the second embodiment of the present invention;
FIG. 9 is a longitudinal cross-sectional view showing a manipulator and a guide mechanism according to the second embodiment of the present invention;
FIG. 10 is a perspective view showing a manipulator according to a first reference embodiment of the present invention;
FIG. 11 is an appearance view showing a rotation restricting mechanism according to a second reference embodiment of the present invention;
FIG. 12 is a transverse cross-sectional view showing the rotation restricting mechanism according to the second reference embodiment of the present invention; and
FIG. 13 is a transverse cross-sectional view showing the rotation restricting mechanism according to the present invention taken along a line XIII-XIII in FIG. 12.

Each embodiment according to the present invention will now be explained hereinafter in detail.

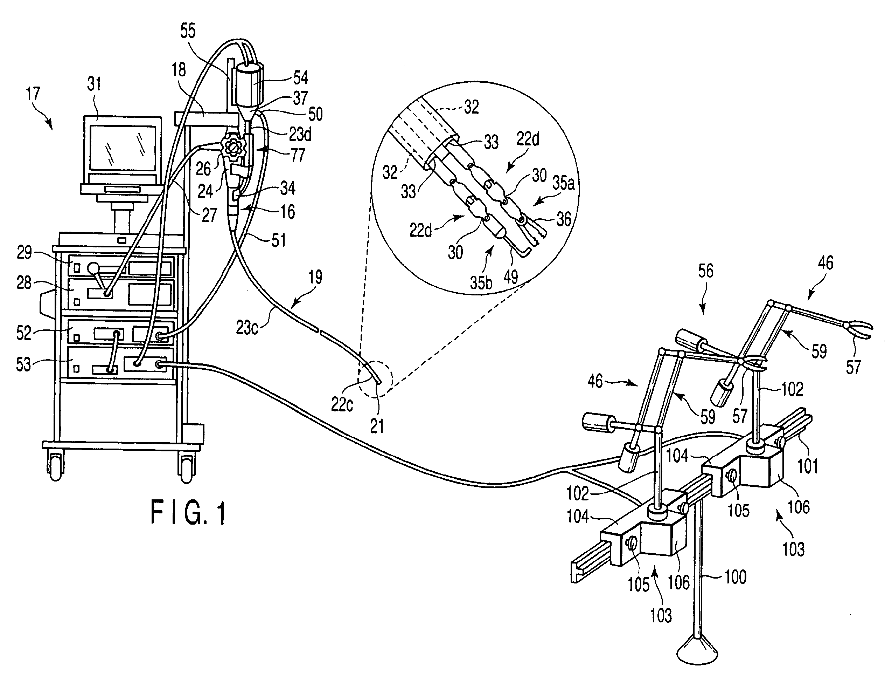

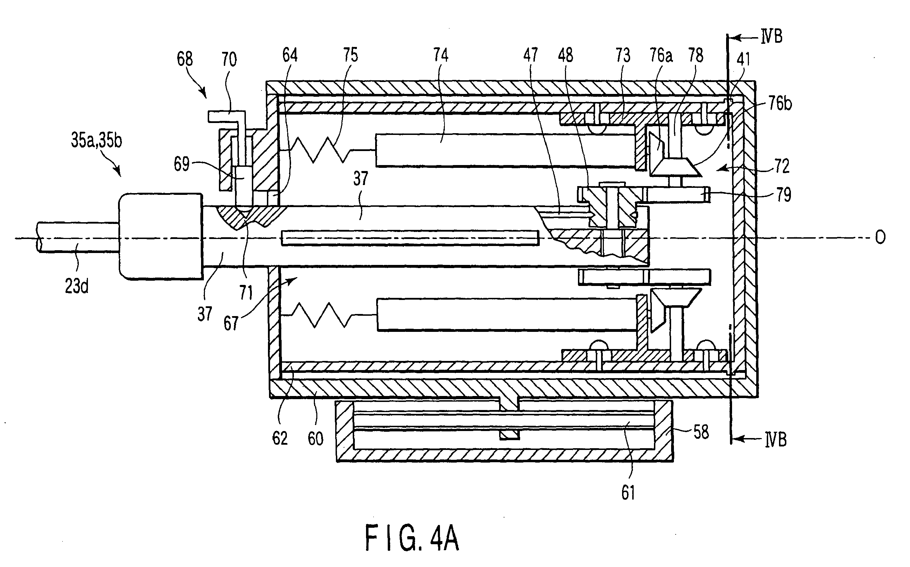

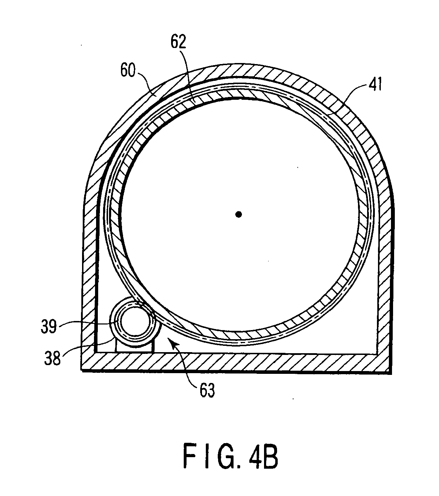

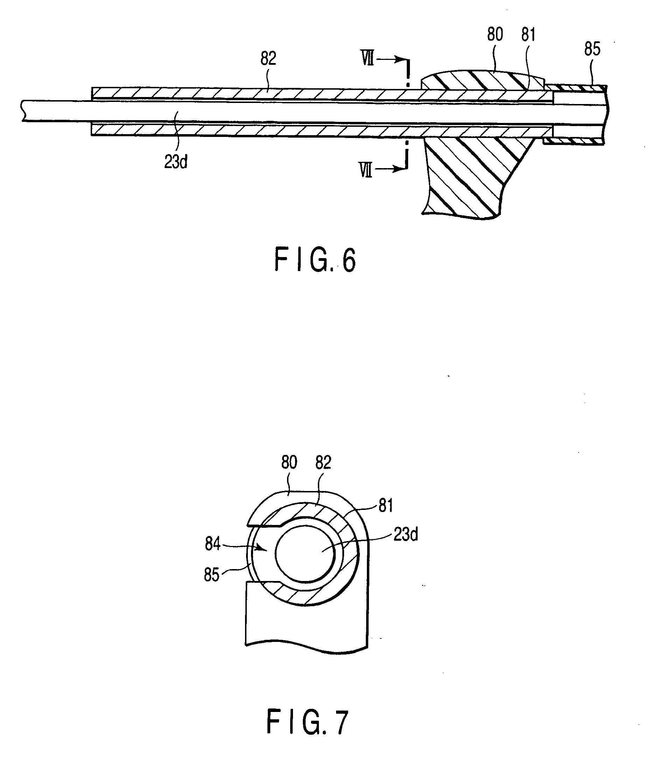

FIGS. 1 to 7 show a first embodiment of the present invention.

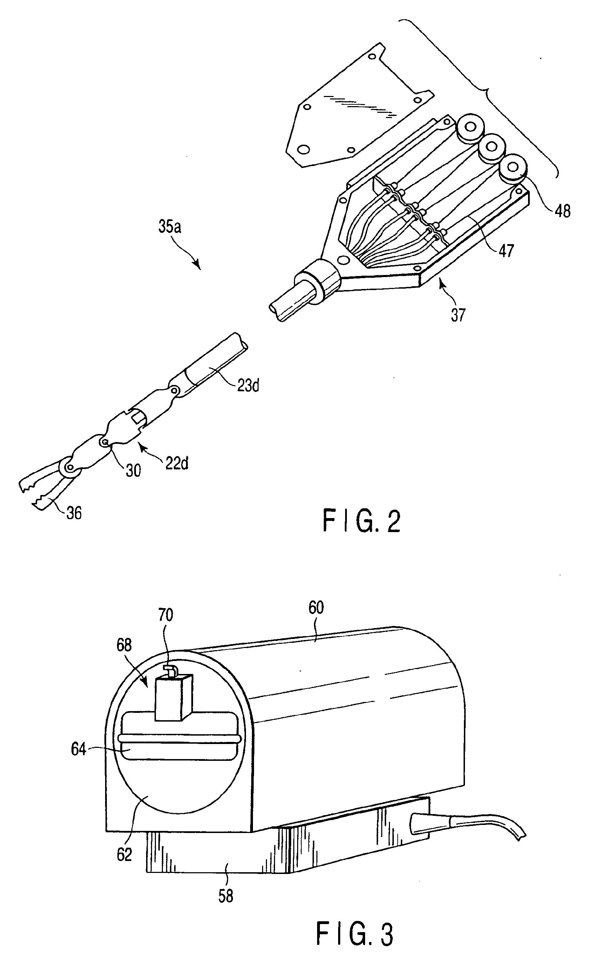

An outline structure of a medical system will now be explained with reference to FIGS. 1 and 2.

In regard to an endoscope 16 as an insertion device, a proximal end portion of the endoscope 16 is supported by an endoscope support arm 18 arranged in a trolley 17. The endoscope 16 includes a long insertion portion 19 to be inserted into a body cavity. In the insertion portion 19, a distal end rigid portion 21, an endoscope bending portion 22c to be operated to be bent, and an endoscope insertion tube portion 23c long and flexible are continuously provided from a distal end side to a proximal end side. An operating portion 24 to be operated by an operator is coupled with a proximal end portion of the insertion portion 19. A bending operation knob 26 to operate the endoscope being portion 22c to be bent and others are arranged in the operating portion 24. A universal cable 27 is extended from the operating portion 24. The universal cable 27 is connected with a video processor 28 and a light source device 29 mounted on the trolley 17. Illumination light generated from the light source device 29 is supplied to an illumination optical system of the distal end rigid portion 21 through a light guide inserted through the endoscope 16, and applied to an observation target. An image pick-up unit within the distal end rigid portion 21 picks up an observation image to generate an image signal, and the image signal is output to the video processor 28 via an image pick-up cable inserted through the endoscope 16. The video processor 28 displays an observation image in a monitor 31. Further, first and second channels 32 are extended through the insertion portion 19. Distal end portions of the first and second channels 32 open at the distal end rigid portion 21 and form first and second protruding openings 33. Proximal end portions of the first and second channels 32 are connected with first and second insertion-into connecters 34 as proximal end opening portions protruding on the operating portion 24.

In regard to a forceps manipulator 35a and an accessory manipulator 35b as a treatment device, in the forceps manipulator 35a, a grasping portion 36 to be actuated to be opened and closed, a bending portion 22d to be actuated to be bent, a insertion tube portion 23d long and flexible, and a coupling unit 37 are continuously provided from a distal end side to a proximal end side. When the coupling unit 37 as a coupling portion is driven to move forward or backward in a longitudinal axis direction of the forceps manipulator 35a, or rotated around the longitudinal axis, the insertion tube portion 23d as a transmitting portion transmits a driving force and the bending portion 22d and the grasping portion 36 as an actuating portion are moved forward or backward, or rotated. Furthermore, respective pairs of operation wires 47 to actuate the grasping portion 36 and respective joint portions 30 of the bending portion 22d are inserted through the bending portion 22d and the insertion tube portion 23d, and led into the coupling unit 37. Each pair of operation wires 47 includes a proximal end portion formed into a continuous annular shape, and is wound around each pulley 48 arranged in the coupling unit 37. When each pulley 48 as a coupling mechanism is driven to rotate, one and the other operation wire 47 of the pair of operation wires 47 as a transmission member are moved forward and backward, and so the grasping portion 36 and each joint portion 30 of the bending portion 22d as an actuation mechanism is actuated.

Moreover, the accessory manipulator 35b includes substantially the same structure as the forceps manipulator 35a. That is, in the accessory manipulator 35b, an electrode portion 49 through which a high-frequency current is to flow, the bending portion 22d as the actuating portion, the insertion tube portion 23d, and the coupling unit 37 are continuously provided from a distal end side to a proximal end side, and the operation wires 47 and the pulleys 48 are arranged in the accessory manipulator 35b. An electrode wire through which a high-frequency current is to flow toward the electrode portion 49 is extended from the electrode portion 49, and inserted through the bending portion 22d and the insertion tube portion 23d and led into the coupling unit 37. A proximal end portion of the electrode wire is connected with an inner end portion of an electrical connecting portion 50 protruding on the coupling unit 37. The electrical connecting portion 50 is connected with a high-frequency output device 52 mounted on the trolley 17 through an electrical cable 51. A high-frequency current flows from the high-frequency output device 52 to the electrode portion 49 through the electrical cable 51, the electrical connecting portion 50 and the electrode wire. The high-frequency output device 52 is connected with a control device 53 mounted on the trolley 17.

In regard to first and second driving units 54 as a driving device, each driving unit 54 is supported by a driving unit support arm 55 arranged in the trolley 17. The coupling unit 37 of the manipulators 35a and 35b inserted through the first and second channels 32 of the endoscope 16 is to be detachably coupled with the first and second driving units 54, respectively. The driving units 54 is to drive the entire coupling unit 37 to move forward and backward in a longitudinal axis direction of the manipulators 35a and 35b and to rotate around the longitudinal axis, and drive the respective pulleys 48 in the coupling unit 37 to rotate. The first and second driving units 54 are connected with the control device 53 mounted on the trolley 17.

In the control device 53, types of the manipulators 35a and 35b coupled with the respective driving units 54 can be input. In this embodiment, it is possible to input information indicating that a manipulator coupled with each driving unit 54 is the forceps manipulator 35a or the accessory manipulator 35b.

An operation device 56 is connected with the control device 53. First and second master arms 46 are provided in the operation device 56. In the first and second master arms 46, first and second openable and closable opening and closing portions 57 are movably supported by first and second arm portions 59, respectively. When information indicating that a manipulator coupled with the first driving unit 54 is the forceps manipulator 35a has been input, the control device 53 controls the first driving unit 54 to move, and open or close the grasping portion 36 of the forceps manipulator 35a in accordance with a moving operation and an opening or closing operation for the first opening and closing portion 57. On the other hand, when information indicating that a manipulator coupled with the first driving unit 54 is the accessory manipulator 35b has been input, the control device 53 controls the first driving unit 54 to move the electrode portion 49 of the accessory manipulator 35b in accordance with a moving operation for the first opening and closing portion 57 and also controls the high-frequency output device 52 such that a high-frequency current flows through the electrode portion 49 or is stopped in accordance with an opening or closing operation for the first opening and closing portion 57. Likewise, the control device 53 actuates the manipulators 35a and 35b coupled with the second driving unit 54 in accordance with an operation for the second opening and closing portion 57.

Each driving unit 54 will now be explained in detail with reference to FIGS. 2 to 4B.

The coupling unit 37 of the manipulators 35a and 35b has a flat rectangular parallelepiped shape extending in the longitudinal axis direction of the manipulators 35a and 35b. Additionally, a rotation axis of each pulley 48 arranged in the coupling unit 37 extends to be perpendicular to the coupling unit 37.

In the driving unit 54, a forward and backward movement housing 60 is mounted on a pedestal 58. An forward and backward movement driving unit 61 as a driving mechanism, e.g., a ball screw is interposed between the pedestal 58 and the forward and backward movement housing 60, and the forward and backward movement housing 60 is movable forward and backward with respect to the pedestal 58 in a direction of a central axis O of the driving unit 54. A rotation housing 62 is accommodated in the forward and backward movement housing 60. A rotation driving unit 63 as a driving mechanism is interposed between the forward and backward movement housing 60 and the rotation housing 62, and the rotation housing 62 is rotatable around the central axis O of the driving unit 54 with respect to the forward and backward movement housing 60. That is, a rotation motor 38 is fixed on an inner surface of the forward and backward movement housing 60, an output axis portion of the rotation motor 38 is extended in parallel with the direction of the central axis O of the driving unit 54, and a first rotation spur gear 39 is coupled with the output axis portion. A second rotation spur gear 41 is extended on an entire circumference of an outer peripheral portion of the rotation housing 62. The first and second rotation spur gears 39 and 41 tooth with each other, and a rotation driving force of the rotation motor 38 is transmitted to the rotation housing 62 via the first and second rotation spur gears 39 and 41. An insertion and removal hole 64 is formed at a forward side end portion of the rotation housing 62, and a coupling unit accepting portion 67 as a coupling accepting portion is arranged in the rotation housing 62. The coupling unit 37 of each of the manipulators 35a and 35b is to be inserted into and removed from the coupling unit accepting portion 67 in the direction of the central axis O of each driving unit 54 via the insertion and removal hole 64, and a longitudinal axis of the coupling unit 37 inserted in the coupling unit accepting portion 67 matches with the central axis O of the driving unit 54. Further, an engaging unit 68 is arranged near the insertion and removal hole 64 on an outer surface of the rotation housing 62. In the engaging unit 68, an engaging member 69 is supported slidablely in a radial direction of the driving unit 54 and urged toward the inner side in the radial direction. An operation member 70 to operate the engaging member 69 is coupled with the engaging member 69. On the other hand, an engaging concave portion 71 is formed at a distal end portion of the coupling unit 37 of each of the manipulators 35a and 35b. When the coupling unit 37 is inserted into the coupling unit accepting portion 67, the engaging member 69 is engaged with the engaging concave portion 71, and the rotation housing 62 and the coupling unit 37 is engaged with each other. Furthermore, when the coupling unit 37 is removed from the coupling unit accepting portion 37, the engaging member 69 is operated by the operation member 70 and the engagement between the engaging member 69 and the engaging concave portion 71 is released, and so the engagement between the rotation housing 62 and the coupling unit 37 is released.

A pulley driving unit 72 as a driving coupling mechanism to drive each pulley 48 in the coupling unit 37 inserted in the coupling unit accepting portion 67 is arranged in the rotation housing 62. That is, a motor base 73 is arranged on a backward side within the rotation housing 62, and the motor base 73 is movable forward and backward within a predetermined range with respect to the rotation housing 62 in parallel with the direction of the central axis O of the driving unit 54. An actuation motor 74 is arranged on the forward side of the motor base 73. An output axis portion of the actuation motor 74 extends toward the backward side in parallel with the central axis O of the driving unit 54, and a first bevel gear 76a is coupled with an end portion of the output axis portion. A rotation axis portion 78 protrudes on the backward side of the motor base 73. The rotation axis portion 78 is extended perpendicularly to the coupling unit 37 inserted in the coupling unit accepting portion 67 and is rotatable around its own central axis. A second bevel gear 76b toothed with the first bevel gear 76a is coupled with an intermediate portion of the rotation axis portion 78, and an actuation spur gear 79 is coupled with a terminal end portion of the rotation axis portion 78. The actuation spur gear 79 is toothed with the pulley 48 in the coupling unit 37 inserted in the coupling unit accepting portion 67. The pulley driving unit 72 is constantly urged forward by a tension spring 75. That is, the pulley driving unit 72 is urged in a direction opposite to an inserting direction of the coupling unit 37, and when the coupling unit 37 is inserted into the coupling unit accepting portion 67, each pulley 48 in the coupling unit 37 is toothed assuredly with the actuation spur gear 79 of the pulley driving unit 72. A rotation driving force of the actuation motor 74 is transmitted to the pulley 48 via the first bevel gear 76a, the second bevel gear 76b, and the actuation spur gear 79.

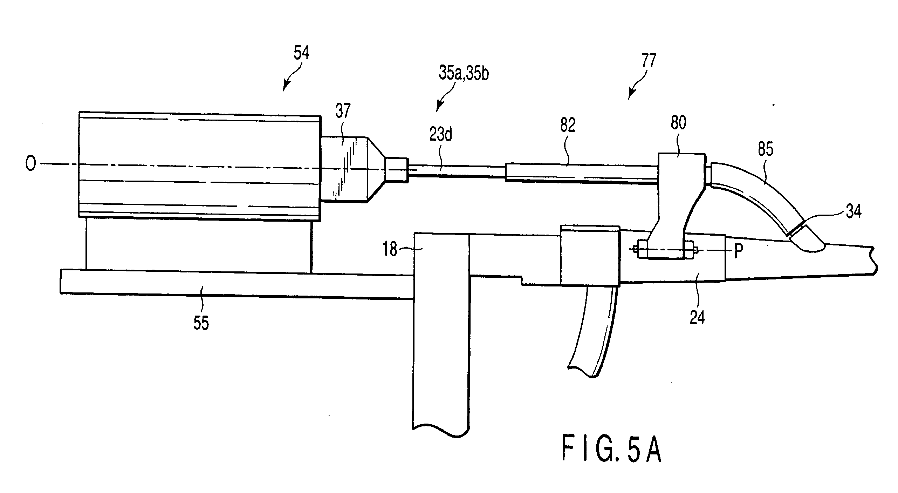

A guide mechanism 77 will now be explained in detail with reference to FIGS. 5A to 7.

The driving unit 54 is arranged such that the central axis O of the driving unit 54 is parallel to a longitudinal axis of the endoscope 16. The first and second driving units 54 are arranged side by side such that the first and second driving units 54 are arranged on the same side as the first and second insertion-into connecters 34 with respect to the longitudinal axis of the endoscope 16, respectively. First and second guide mechanisms 77 are arranged side by side between the first and second insertion-into connecters 34 and the first and second driving units 54, respectively.

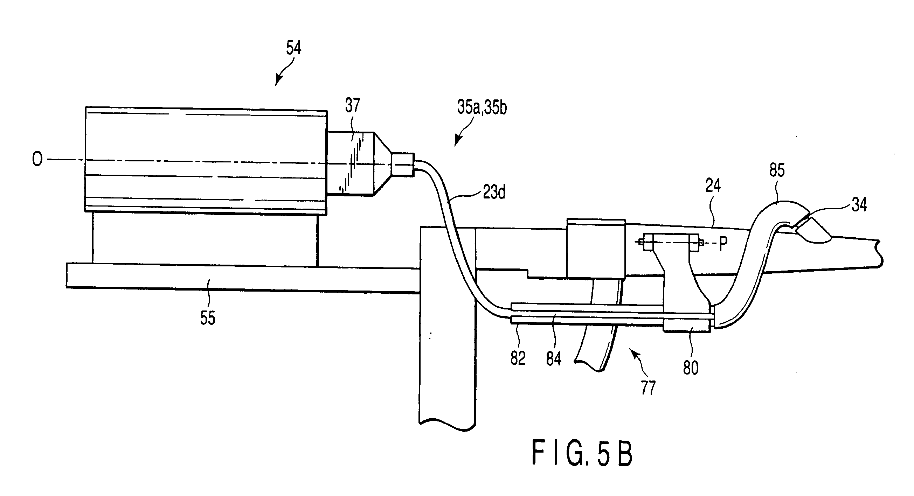

In the guide mechanism 77, a support arm portion 80 as a retreat support mechanism is provided pivotally on a side surface of the operating portion 24 of the endoscope 16. A terminal end portion of the support arm portion 80 is rotatable around a rotational axis P parallel to the longitudinal axis of the endoscope 16. A support hole 81 is extended at the terminal end portion of the support arm portion 80 in parallel with the longitudinal axis direction of the endoscope 16. A guide pipe 82 having a circular tube shape as a guide member is inserted through the support hole 81, and the guide pipe 82 is fixed to the support arm portion 80 and arranged in parallel with the longitudinal axis direction of the endoscope 16. The guide pipe 82 is formed of a material harder than a material of a insertion tube forming the insertion tube portion 23d of the manipulator 35a or 35b. That is, the insertion tube of the manipulator 35a or 35b is formed of, e.g., a fluorocarbon resin, whereas the guide pipe 82 is formed of, e.g., a metal, preferably stainless or brass, or a resin, preferably a polyeth-eretherketon resin, polycarbonate, or polypropylene, or a mixture of these. The guide pipe 82 is rotatable between a guide position (see FIG. 5A) and a retreat position (see FIG. 5B) by the support arm portion 80. At the guide position, a central axis of the guide pipe 82 matches with the central axis O of the driving unit 54. At the retreat position, the guide pipe 82 is arranged symmetrically with the guide position with respect to the rotational axis of the support arm portion 80. Further, in the guide pipe 82, an attachment and detachment groove 84 as an attachment and detachment portion having a notched groove shape is extended over the entire length of the guide pipe 82 in the longitudinal axis direction. A notched groove is also formed in the support arm portion 80 at a position facing the attachment and detachment groove 84 of the guide pipe 82. A width of the attachment and detachment groove 84 is slightly smaller than an outer diameter of the insertion tube portion 23d of the manipulator 35a or 35b. A soft guide tube 85 formed of, e.g., polytetrafluoroethylene is disposed between a distal end portion of the guide pipe 82 and a protruding end portion of the insertion-into connecter 34.

A method of using the medical system according to this embodiment will now be explained.

When using the medical system, the insertion portion 19 of the endoscope 16 is inserted into a body cavity. In the guide mechanism 77, the guide pipe 82 is arranged at the retreat position, and one end portion of the guide tube 85 is connected with the insertion-into connecter 34 of the endoscope 16. The coupling unit 37 of the manipulator 35a or 35b is inserted into the coupling unit accepting portion 67 of the driving unit 54, the rotation housing 62 is engaged with the coupling unit 37, and the manipulator 35a or 35b is connected with each driving unit 54. The distal end side of each of the manipulators 35a and 35b is inserted into the corresponding insertion-into connecter 34 through the guide tube 85, inserted through the channel 32, and protruded from the protrusion opening 33 of the distal end rigid portion 21 of the endoscope 16. Subsequently, the guide pipe 82 is moved from the retreat position to the guide position, the insertion tube portion 23d of each of the manipulators 35a and 35b is disposed in the guide pipe 82 through the attachment and detachment groove 84 of the guide pipe 82, and the other end portion of the guide tube 85 is connected with the distal end portion of the guide pipe 82. Further, information indicating that which one of the forceps manipulator 35a and the accessory manipulator 35b is a manipulator connected with each of the first and second driving units 54 is appropriately input to the control device 53.

Subsequently, the first and second opening and closing portions 57 of the first and second master arms 46 of the operation device 56 are operated while an observation image of the endoscope 16 displayed in the monitor 31 is observed, and whereby, the grasping portion 36 of the forceps manipulator 35a is moved, and opened and closed to grasp and support the living tissue, and a high-frequency current flows through the electrode portion 49 of the accessory manipulator 35b, the electrode portion 49 is moved and brought into contact with a living tissue to incise the living tissue. At this time, each driving unit 54 moves forward and backward, and rotates the coupling unit 37 of the manipulator 35a or 35b, and a forward and backward movement driving force and a rotation driving force applied to the coupling unit 37 are transmitted to the bending portion 22d and the grasping portion 36 or the electrode portion 49 by the insertion tube portion 23d, the bending portion 22d and the grasping portion 36 or the electrode portion 49 are moved forward and backward, and rotated. Furthermore, the pulley driving unit 72 of each driving unit 54 rotates each pulley 48, one and the other operation wire 47 of the pair of operation wires 47 are moved forward and backward, and so the grasping portion 36 and each joint portion 30 of the bending portion 22d are actuated. Here, since the insertion tube portion 23d is supported and guided by the hard guide pipe 82 between the coupling unit 37 coupled with the coupling unit accepting portion 67 and the guide tube 85, the insertion tube portion 23d can assuredly transmit the forward and backward movement driving force, and the rotation driving force. Moreover, each pair of operation wires 47 inserted through the insertion tube portion 23d can assuredly transmit the driving force.

When removing each of the manipulators 35a and 35b, the support arm portion 80 is rotated and so the guide pipe 82 is moved to the retreat position. The insertion tube portion 23d of each of the manipulators 35a and 35b is removed from the guide pipe 82 via the attachment and detachment groove 84 while the other end portion of the guide tube 85 is removed from the distal end portion of the guide pipe 82. Additionally, the distal end side of each of the manipulators 35a and 35b is removed from the endoscope 16 and the guide tube 85. Then, engagement between the coupling unit 37 and the rotation housing 62 is released, the coupling unit 37 is removed from the coupling unit accepting portion 67, and each of the manipulators 35a and 35b is separated from each driving unit 54.

Therefore, the medical system according to this embodiment demonstrates the following effect.

In the medical system according to this embodiment, the guide pipe 82 harder than the insertion tube portion 23d supports and guides the insertion tube portion 23d between the coupling unit 37 coupled with the coupling unit accepting portion 67 and the insertion-into connecter 34. Therefore, the insertion tube portion 23d can assuredly transmit the forward and backward movement driving force, and the rotation driving force applied to the coupling unit 37 by the driving unit 54 to the bending portion 22d and the grasping portion 36 or the electrode portion 49, and so the bending portion 22d and the grasping portion 36 or the electrode portion 49 can be assuredly moved forward and backward, and rotated. Further, the operation wires 47 inserted through the insertion tube portion 23d can assuredly transmit the driving force applied to the pulleys 48 to the bending portion 22d and the grasping portion 36, and so the bending portion 22d and the grasping portion 36 is assuredly actuated.

Furthermore, since the entire guide mechanism 77 can be retreated from the space between the coupling unit 37 coupled with the coupling unit accepting portion 67 and the insertion-into connecter 34, the space where the coupling unit 37 is attached to and detached from the coupling unit accepting portion 67 can be sufficiently assured, and so the attachment and detachment operation for the coupling unit 37 with respect to the coupling unit accepting portion 67 can be carried out easily. Moreover, the insertion tube portion 23d can be attached to and detached from the guide pipe 82 in the radial direction of the guide pipe 82 through the attachment and detachment groove 84 of the guide pipe 82, and so the attachment and detachment operation for the insertion tube portion 23d with respect to the guide pipe 82 can be carried out easily.

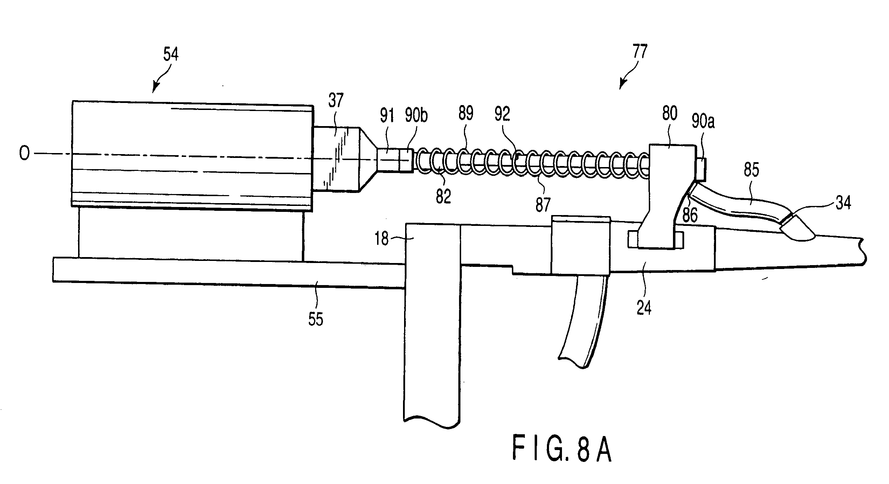

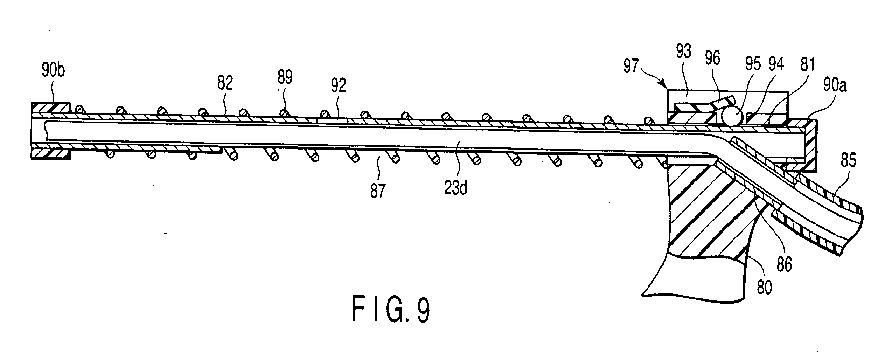

FIGS. 8A to 9 show a second embodiment of the present invention.

In the guide mechanism 77 according to this embodiment, the support arm portion 80 as a follow-up support mechanism and a retreat support mechanism is fixed to the operating portion 24 of the endoscope 16. An insertion-through connecter 86 is embedded in the support arm portion 80. An inner end portion of the insertion-through connecter 86 protrudes toward the inside of the support hole 81 of the support arm portion 80, and an outer end portion of the insertion-through connecter 86 protrudes from the support arm portion 80 and is arranged to face the insertion-into connecter 34 of the endoscope 16. The guide tube 85 is disposed between the insertion-through connecter 86 and the insertion-into connecter 34. The insertion-into connecter 34, the guide tube 85, and the insertion-through connecter 86 form a proximal end opening portion. The guide pipe 82 is inserted through the support hole 81 of the support arm portion 80 such that the guide pipe 82 is movable forward and backward along a longitudinal axis direction of the endoscope 16. In the guide pipe 82, an escape groove 87 as a follow-up escape portion and a retreat escape portion having a notched groove shape is extended over the entire length of the guide pipe 82 along the axial direction on the endoscope 16 side. An inner end portion of the insertion-through connecter 86 is inserted into the escape groove 87 of the guide pipe 82 and the guide pipe 82 and the insertion-through connecter 86 are not interfered with each other in the forward and backward movement of the guide pipe 82.

A distal end stopper 90a and a proximal end stopper 90b as a contact portion having a cylindrical shape are fit on and fixed at a distal end portion and a proximal end portion of the guide pipe 82, respectively. A coil spring 89 as an urging mechanism and a fall-preventing member is compressed and arranged between the support arm portion 80 and the proximal end stopper 90b of the guide pipe 82, and the coil spring 89 is fit on the guide pipe 82. The guide pipe 82 is urged toward the proximal end side with respect to the support arm portion 80 by the coil spring 89. When the distal end stopper 90a of the guide pipe 82 comes into contact with the support arm portion 80, the movement of the guide pipe 82 toward the proximal end side is restricted. On the other hand, at a distal end portion of the coupling unit 37 of each of manipulators 35a and 35b, a cylindrical stopper accepting portion 91 as a contact accepting portion is coaxially fit on and fixed to the insertion tube portion 23d. The proximal end stopper 90b of the guide pipe 82 is to be brought into contact with the stopper accepting portion 91 of the coupling unit 37.

Further, a holding unit 97 to hold the guide pipe 82 at the most distal end retreat position is arranged in the guide mechanism 77. That is, in the guide pipe 82, a holding hole 92 pierces in a radial direction at a predetermined position with respect to the axial direction. An accommodation groove 93 is extended in an outer surface portion of the support arm portion 80 along the support hole 81, and an insertion hole 94 is formed between a bottom portion of the accommodation groove 93 and the support hole 81. An holding member 95 is arranged in the insertion hole 94, and the holding member 95 is urged toward the inner side in a radial direction of the support hole 81 by a leaf spring 96. When the guide pipe 82 is not arranged at the retreat position, the holding member 95 is in contact with an outer peripheral surface of the guide pipe 82. When the guide pipe 82 is arranged at the retreat position, the holding hole 92 of the guide pipe 82 is aligned with the insertion hole 94 of the support arm portion 80, and the holding member 95 is held in the holding hole 92 of the guide pipe 82.

The method of using the medical system according to this embodiment will now be explained.

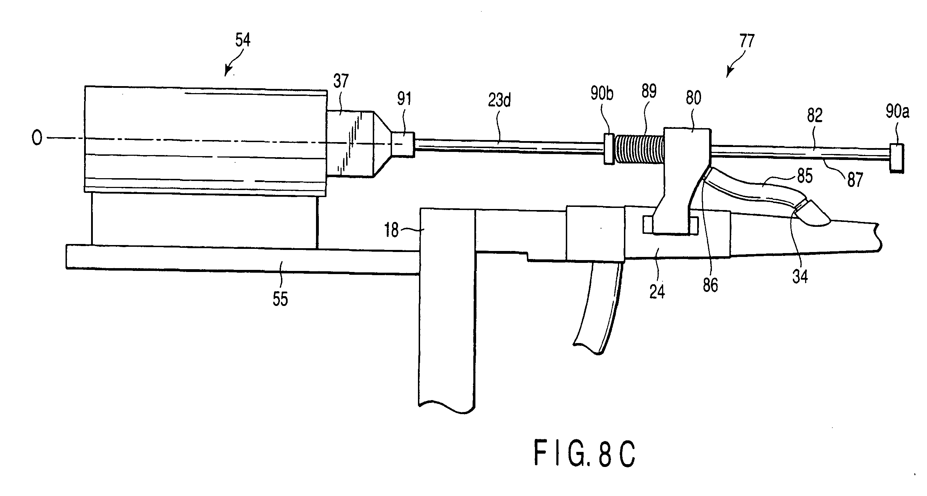

When using the medical system, as shown in FIG. 8C, in the guide mechanism 77, the guide pipe 82 is moved toward the distal end side to reach the retreat position against an urging force of the coil spring 89, the holding member 95 of the support arm portion 80 is held in the holding hole 92 of the guide pipe 82, and so the guide pipe 82 held with respect to the support arm portion 80. Subsequently, each of manipulators 35a and 35b is coupled with each driving unit 54, and the distal end side of each of the manipulators 35a and 35b is inserted into the proximal end portion of the guide pipe 82, and inserted through the guide pipe 82, the insertion-through connecter 86 and the guide tube 85, and inserted into the insertion-into connecter 34 of the endoscope 16, and inserted through the channel 32, and protruded from the protrusion opening 33 of the distal end rigid portion 21. Then, the guide pipe 82 is moved to the proximal end side, and the holding member 95 is pushed out from the holding hole 92, and so a holding state of the guide pipe 82 and the support arm portion 80 is released. As a result, as shown in FIG. 8A, the guide pipe 82 is moved toward the proximal end side by the urging force of the coil spring 89, and the proximal end stopper 90b of the guide pipe 82 is brought into contact with the stopper accepting portion 91 of the coupling unit 37 of each of the manipulators 35a and 35b.

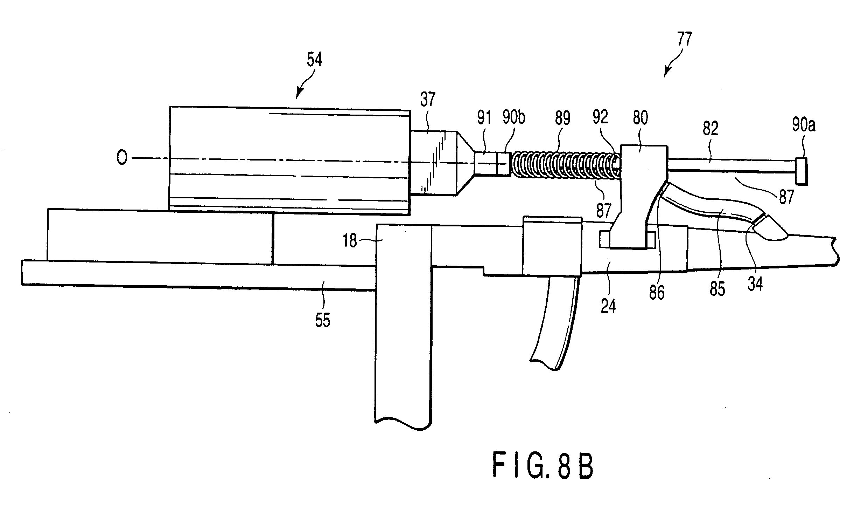

Like the first embodiment, the operation device 56 is operated and so each of the manipulators 35a and 35b is actuated. Here, as shown in FIG. 8B, when the coupling unit 37 is moved forward, the stopper accepting portion 91 of the coupling unit 37 moves the proximal end stopper 90b of the guide pipe 82 toward the distal end side against the urging force of the coil spring 89, and the guide pipe 82 is moved toward the distal end side. On the other hand, when the coupling unit 37 is moved backward, the guide pipe 82 is moved toward the proximal end side in a state where the proximal end stopper 90b of the guide pipe 82 is in contact with the stopper accepting portion 91 of the coupling unit 37 by the urging force of the coil spring 89. In this manner, the guide pipe 82 is moved forward and backward in accordance with the forward movement and backward movement of the coupling unit 37. As explained above, during driving of the coupling unit 37, the proximal end stopper 90b of the guide pipe 82 is constantly in contact with the stopper accepting portion 91 of the coupling unit 37, and a space where the insertion tube portion 23d is not supported by the guide pipe 82 is not formed between the coupling unit 37 side end portion of the guide pipe 82 and the coupling unit 37.

When removing each of the manipulators 35a and 35b, the guide pipe 82 is arranged at the retreat position like attachment of each manipulator, and the distal end side of each of the manipulators 35a and 35b is removed from the endoscope 16, the guide tube 85, and the guide mechanism 77.

Therefore, the medical system according to this embodiment demonstrates the following effect.

In the medical system according to this embodiment, since the guide pipe 82 is moved forward and backward in accordance with the forward movement and backward movement of the coupling unit 37 in a state where the proximal end stopper 90b of the guide pipe 82 is in contact with the stopper accepting portion 91 of the coupling unit 37, the space where the insertion tube portion 23d is not supported by the guide pipe 82 is not formed between the coupling unit 37 side end portion of the guide pipe 82 and the coupling unit 37, and so the insertion tube portion 23d and each operation wire 47 can more assuredly transmit the driving force. Furthermore, when at least a part of the guide pipe 82 is retreated in a forward and backward movement direction of the coupling unit 37 from the space between the coupling unit accepting portion 67 and the support arm portion 80 and the guide pipe 82 is arranged and held at the retreat position, the space where the coupling unit 37 is to be attached to and detached from the coupling unit accepting portion 67 is assured without holding the guide pipe 82 by an operator against the urging force of the coil spring 89, and so the attachment and detachment operation for the coupling unit 37 with respect to the coupling unit accepting portion 67 can be carried out easily.

Moreover, although a width of the escape groove 87 of the guide pipe 82 is slightly larger than an outside diameter of the insertion-through connecter 86 and larger than an outside diameter of the insertion tube portion 23d in order that the guide pipe 82 and the insertion-through connecter 86 do not interfere with each other, the coil spring 89 is fit on the guide pipe 82 and so the insertion tube portion 23d is prevented from falling off the escape groove 87 of the guide pipe 82 by the coil spring 89.

A third embodiment according to the present invention will now be explained.

In the guide mechanism 77 according to this embodiment, the support arm portion 80 is rotatable, like the first embodiment, in the guide mechanism 77 according to the second embodiment. In a medical system according to this embodiment, since the entire guide mechanism 77 can be retreated from a space between the coupling unit 37 coupled with the coupling unit accepting portion 67 and the insertion-into connecter 34, a space where the coupling unit 37 is to be attached to and detached from the coupling unit accepting portion 67 can be sufficiently assured as compared with the medical system according to the second embodiment.

In the foregoing embodiment, an endoscope is used as an insertion device, but an over-tube and others can be used. Further, a pipe-like member is used as a guide member, but a bent-plate-like member or a square-bar-like member wherein a guide groove through which an insertion tube portion of a manipulator is guided is extended may be used, for example.

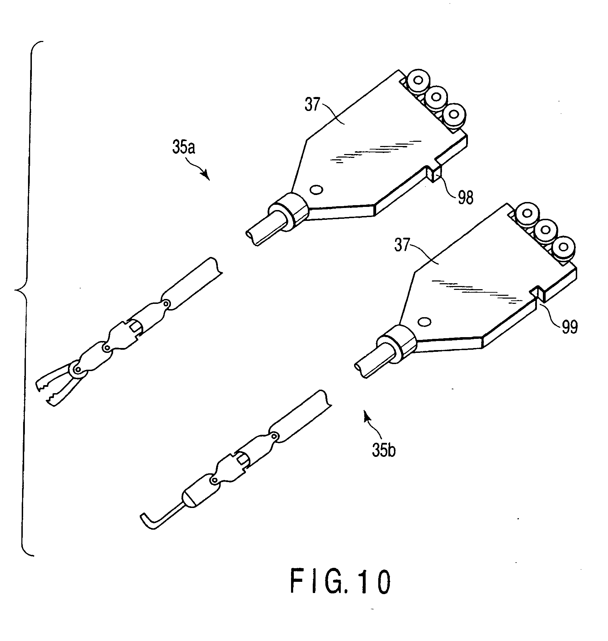

FIG. 10 shows a first reference embodiment according to the present invention.

Referring to FIGS. 1 and 10, an identification mechanism to identify a type of each of manipulators 35a and 35b is arranged in each of the manipulators 35a and 35b and each driving unit 54. In this reference embodiment, as the identification mechanism, a mechanical identification mechanism is used. That is, a convex portion 98 is formed on a coupling unit 37 of a forceps manipulator 35a, and a concave portion 99 is formed on the coupling unit 37 of the accessory manipulator 35b. A judgment mechanism to determine which one of the convex portion 98 and the concave portion 99 is formed on the coupling unit 37 is arranged in each driving unit 54. The driving unit 54 outputs a judgment signal indicative of a judgment result to the control device 53. The control device 53 determines which one of the forceps manipulator 35a and the accessory manipulator 35b is coupled with the driving unit 54 based on the judgment signal, and stores a determined result. The control device 53 controls each of the first and second driving units 54 in accordance with an operation for each of the first and second master arms 46 based on the stored type of the manipulator.

In the medical system according to this reference embodiment, an operator does not have to input information indicating that a manipulator coupled with each driving unit 54 is the forceps manipulator 35a or the accessory manipulator 35b, and an input operation can be omitted. Furthermore, it is possible to avoid occurrence of an erroneous operation of the medical system caused due to an erroneous input of a type of each of the manipulators 35a and 35b.

In this reference embodiment, although the mechanical identification mechanism is used as the identification mechanism, a software type identification mechanism that uses an IC tag and so or an electrical identification mechanism that uses electrical resistance may be adopted, for example.

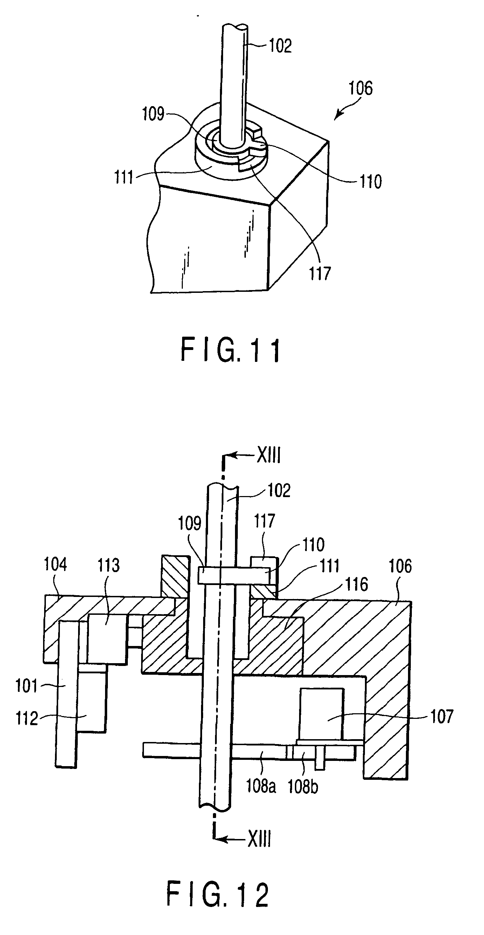

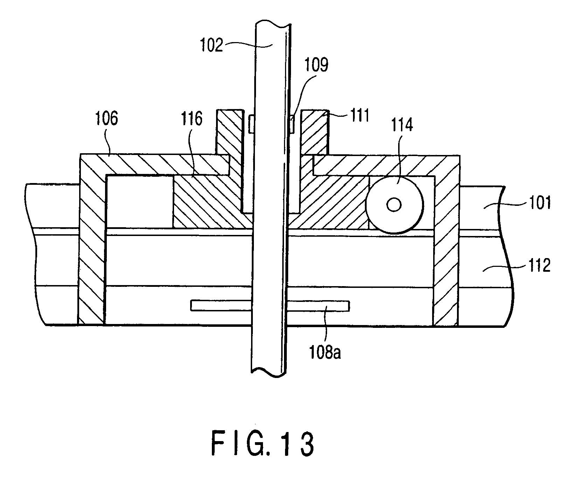

FIGS. 11 to 13 show the second reference embodiment according to the present invention.

Referring to FIG. 1 and FIGS. 11 to 13, the operation device 56 according to this reference embodiment includes a columnar support portion 100 arranged on, e.g., a floor. A top portion of the columnar support portion 100 is coupled with a central portion of a guide rail 101. The guide rail 101 is extended in a horizontal direction. Slide portions 104 of first and second master bases 103 are disposed at both end side portions of the guide rail 101 and slidable in a longitudinal axis direction of the guide rail 101. A transverse cross-section of each slide portion 104 has a concave shape, and each slide portion 104 is mounted on the guide rail 101. Handles 105 are arranged on one side wall of each slide portion 104. When the handles 105 is screwed and the guide rail 101 is held by the handles 105 and the other side wall of the slide portion 104, the slide portion 104 is fixed to the guide rail 101.

A base portion 106 of each of the first and second master bases 103 supports a proximal end arm 102 of each of the first and second master arms 46. The proximal end arm 102 is arranged in a vertical direction and is rotatable around a central axis of the proximal end arm 102 with respect to the master base 103.

The master base 103 includes an encoder 107 to detect a rotation amount of the proximal end arm 102 with respect to the master base 103. A first detection spur gear 108a coupled with the proximal end arm 102 is toothed with a second detection spur gear 108b coupled with a rotation axis portion of the encoder 107. To improve a resolution for detection of a rotation amount, a gear ratio of the second detection spur gear 108b with respect to the first detection spur gear 108a is larger than 1. A rotation range of the proximal end arm 102 must be restricted to a rotation range obtained by multiplying 360° by an inverse number of the gear ratio in order to set a rotation range of the rotation axis portion of the encoder to 360° or below. That is, a rotation stopper 109 is coupled with the proximal end arm 102. The rotation stopper 109 is formed of an annular portion coaxially fit on and fixed to the proximal end arm 102 and a protruding portion 110 protruding from the annular portion toward the outside in a radial direction. On the other hand, a rotation stopper accepting portion 111 is arranged on the master base 103. The proximal end arm 102 and the annular portion of the rotation stopper 109 are coaxially arranged in a cylindrical wall of the rotation stopper accepting portion 111, and the protruding portion 110 of the rotation stopper 109 is inserted into a notched portion 117 extended in the cylindrical wall in a circumferential direction. When the protruding portion 110 comes into contact with both end walls of the notched portion 117, rotation of the proximal end arm 102 with respect to the master base 103 is restricted. A central angle of the notched portion 117 is set so that the rotation range of the proximal end arm 102 is restricted to the above-explained rotation range.

From a viewpoint of operability of the operation device 56, it is preferable to appropriately change the rotation range of the proximal end arm 102 with respect to the master base 103 in accordance with arrangement of the master base 103 with respect to the guide rail 101, for example, such that the center of the rotation range constantly faces an operator. That is, a rack 112 is extended in the guide rail 101 in the axial direction of the guide rail 101. In the rack 112, many tooth portions are arranged side by side along the axial direction of the guide rail 101 on a top portion side for the longitudinal axis direction of proximal end arm 102. A pinion 113 is toothed with the rack 112. The pinion 113 is supported by a base portion 106 to be rotatable around a rotation axis perpendicular to the longitudinal axis direction of the guide rail 101 and the central axis direction of the proximal end arm 102. A worm gear 114 is coaxially coupled with the pinion 113. A worm wheel 116 is toothed with the worm gear 114. The worm wheel 116 is supported by the base portion 106 coaxially with the base end arm 102 to be rotatable around the central axis of the proximal end arm 102, and the proximal end arm 102 is inserted into a central opening of the worm wheel 116. A stopper accepting portion 91 is coaxially coupled with the worm wheel 116 on the top portion side for the longitudinal axis direction of the proximal end arm 102. When the master base 103 is moved with respect to the guide rail 101, the pinion 113 toothed with the rack 112 of the guide rail 101 rotates, rotation is transmitted to the pinion 113, the worm gear 114, and the worm wheel 116 and so the rotation stopper accepting portion 111 coupled with the worm wheel 116 is rotated and the center of the rotation range of the proximal end arm 102 is changed. With such a mechanism, the rotation range of the proximal end arm 102 of the master arm 46 can be appropriately set in accordance with arrangement of the master base 103 with respect to the guide rail 101. Furthermore, rotation is apt to be transmitted from the worm gear 114 to the worm wheel 116, but rotation is hardly transmitted from the worm wheel 116 to the worm gear 114. Therefore, even if the proximal end arm 102 is rotated with respect to the master base 103 by an operation for the master arm 46 and the protruding portion 110 of the rotation stopper 109 of the proximal end arm 102 comes into contact with the end wall of the notched portion 117 of the rotation stopper accepting portion 111, rotation is not transmitted from the worm wheel 116 coupled with the rotation stopper accepting portion 111 to the worm gear 114, the rotation stopper accepting portion 111 is not rotated, and rotation is not transmitted to the pinion 113.

According to the medical system in this reference embodiment, in the operation device 56, a resolution for detection of a rotation amount of the proximal end arm 102 with respect to the master base 103 is improved, and the rotation range of the proximal end arm 102 is appropriately set in accordance with arrangement of the master base 103 with respect to the guide rail 101, and so operability of the operation device 56 is improved.

## Claims

1. A medical system comprising:
an insertion device (16) having a longitudinal axis, configured to be inserted into a body cavity and including a channel (32) extending between a distal end portion of the insertion device (16) and a proximal end portion thereof and a proximal end opening portion (34; 34, 85, 86) connected with a proximal end portion of the channel (32); and
a treatment device (35a; 35b) configured to be inserted into the channel (32) from the proximal end opening portion (34; 34, 85, 86) and to be inserted through the channel (32), and including an actuating portion (22d, 36; 22d) provided at a distal end portion of the treatment device (35a; 35b), a coupling portion (37) provided at a proximal end portion of the treatment device (35a; 35b) and provided outside of the insertion device (16), and a transmitting portion (23d) extending between the actuating portion (22d, 36; 22d) and the coupling portion (37) and configured to transmit a driving force applied to the coupling portion (37) to the actuating portion (22d, 36; 22d),
**characterized by** further comprising:
a driving device (54) arranged on a same side as the proximal end opening portion (34; 34, 85, 86) with respect to the longitudinal axis of the insertion device (16) and having a central axis (O) parallel to the longitudinal axis of the insertion device (16), the driving device (54) further including a coupling accepting portion (67) configured to be coupled with the coupling portion (37) and a driving mechanism (61; 63) configured to drive the coupling portion (37) coupled with the coupling accepting portion (67) to move in an axial direction of the central axis (O) and to rotate about the central axis (O); and
a guide mechanism (77) including a tubular guide member (82) which is harder than the transmitting portion (23d) and through which the transmitting portion (23d) is configured to be inserted, and a supporting portion (80) which is connected to the insertion device (16) and supports the tubular guide member (82), the tubular guide member (82) being configured to guide and support the transmitting portion (23d), and the supporting portion (80) being configured to arrange the guide member (82) in a guide position where the tubular guide member (82) is arranged between the coupling portion (37) coupled with the coupling accepting portion (67) and the proximal end opening portion (34; 34, 85, 86) in the axial direction of the central axis (O) and a central axis of the guide member (82) matching with the central axis (O) of the driving device (54) at the guide position,
wherein
the coupling portion (37) is attachable to and detachable from the coupling accepting portion (67), and
the supporting portion (80) is configured to support the tubular guide member (82) such that the tubular guide member (82) is movable between the guide position and a retreat position where the tubular guide member (82) is retreated from a space between the coupling portion (37) coupled with the coupling accepting portion (67) and the proximal end opening portion (34; 34, 85, 86) toward the axial direction of the central axis (O) or toward a direction about a rotational axis (P) parallel to the longitudinal axis of insertion device (16).

2. The medical system according to claim 1, **characterized in that**
the guide member (82) includes an attachment and detachment portion (84) through which the transmitting portion (23d) is attachable to and detachable from the guide member (82) in a direction crossing a guiding direction along which the transmitting portion (23d) is guided by the guide member (82).

3. The medical system according to claim 1, **characterized in that**
the tubular guide member (82) includes a retreat escape portion (87) to have at least one of the transmitting portion (23d) and the proximal end opening portion (34; 34, 85, 86) escape such that the tubular guide member (82) does not interfere with at least one of the proximal end opening portion (34; 34, 85, 86) and the transmitting portion (23d) extending between the coupling portion (37) coupled with the coupling accepting portion (67) and the proximal end opening portion (34; 34, 85, 86) in regard to the movement of the tubular guide member (82) in the axial direction of the central axis (O) to the retreat position.

4. The medical system according to claim 3, **characterized in that**
the guide mechanism (77) includes a fall-preventing member (89) covering the retreat escape portion (87) from an outer side of the guide member (82).

5. The medical system according to claim 1, **characterized in that**
the supporting portion (80) supports the guide member (82) so as to be movable in the axial direction of the central axis (O)
a guide mechanism (77) includes an urging mechanism (89) urging the guide member (82) toward a coupling accepting portion (67) side in the axial direction of the central axis (O),
the guide member (82) includes a contact portion (90b) provided at a coupling accepting portion (67) side end portion, and
the coupling portion (37) includes a contact accepting portion (91) arranged at the proximal end portion of the transmitting portion (23d) and with which the contact portion (90b) is configured to come into contact.

6. The medical system according to claim 5, **characterized in that**
the guide member (82) includes a follow-up escape portion (87) to have at least one of the transmitting portion (23d) and the proximal end opening portion (34; 34, 85, 86) escape such that the guide member (82) does not interfere with at least one of the proximal end opening portion (34; 34, 85, 86) and the transmitting portion (23d) extending between the coupling portion (37) coupled with the coupling accepting portion (67) and the proximal end opening portion (34; 34, 85, 86) in regard to the movement of the guide member (82) in the axial direction of the central axis (O).

7. The medical system according to claim 6, **characterized in that**
the guide mechanism (77) includes a fall-preventing member (89) covering the follow-up escape portion (87) from the outer side of the guide member (82).

8. The medical system according to claim 1, **characterized in that**
the supporting portion (80) supports the guide member (82) so as to be movable in the axial direction of the central axis (O),
the guide mechanism (77) includes an urging mechanism (89) for urging the tubular guide member (82) toward a coupling accepting portion (67) side in the axial direction of the central axis (O), and an holding mechanism (97) configured to hold the tubular guide member (82) at the retreat position,
the tubular guide member (82) includes a contact portion (90b) provided at a coupling accepting portion (67) side end portion, and
the coupling portion (37) includes a contact accepting portion (91) arranged at the proximal end portion of the transmitting portion (23d) and with which the contact portion (90b) is configured to come into contact.

9. The medical system according to claim 1, **characterized in that**
the treatment device (35a; 35b) includes an actuation mechanism (30; 36) provided in the actuating portion (22d, 36; 22d), a coupling mechanism (48) provided in the coupling portion (37), and a transmission member (47) inserted through the transmitting portion (23d), extending between the actuation mechanism (30; 36) and the coupling mechanism (48), and to transmit a driving force applied to the coupling mechanism (48) to the actuation mechanism (30; 36) to actuate the actuation mechanism (30; 36), and
the driving device (54) includes a driving coupling mechanism (48) to drive the coupling mechanism (48).

## Patentansprüche

1. Medizinisches System mit:
einer eine Längsachse aufweisenden Einführvorrichtung (16), die dazu ausgebildet ist, in einen Körperhohlraum eingeführt zu werden und die einen Kanal (32), der sich zwischen einem distalen Endbereich der Einführvorrichtung (16) und einem proximalen Endbereich derselben erstreckt, und einen proximalen Endöffnungsbereich (34; 34, 85, 86) aufweist, der mit einem proximalen Endbereich des Kanals (32) verbunden ist; und
einer Behandlungsvorrichtung (35a; 35b), die dazu ausgebildet ist, vom proximalen Endöffnungsbereich (34; 34, 85, 86) aus in den Kanal (32) hinein und durch den Kanal (32) eingeführt zu werden, und die ein Betätigungsteil (22d, 36; 22d), das an einem distalen Endbereich der Behandlungsvorrichtung (35a; 35b) vorgesehen ist, ein Verbindungsteil (37), das an einem proximalen Endbereich der Behandlungsvorrichtung (35a; 35b) und außerhalb der Einführvorrichtung (16) vorgesehen ist, und einen Übertragungsbereich (23d) aufweist, der sich zwischen dem Betätigungsteil (22d, 36; 22d) und dem Verbindungsteil (37) erstreckt und dazu ausgebildet ist, eine auf das Verbindungsteil (37) ausgeübte Antriebskraft auf das Betätigungsteil (22d, 36; 22d) zu übertragen,
**dadurch gekennzeichnet, dass** es des Weiteren aufweist:
eine Antriebsvorrichtung (54), die bezüglich der Längsachse der Einführvorrichtung (16) auf derselben Seite wie der proximale Endöffnungsbereich (34; 34, 85, 86) angeordnet ist und eine Mittelachse (O) parallel zur Längsachse der Einführvorrichtung (16) besitzt, wobei die Antriebsvorrichtung (54) des Weiteren ein Verbindungsaufnahmeteil (67), das dazu ausgebildet ist, mit dem Verbindungsteil (37) gekoppelt zu werden, und einen Antriebsmechanismus (61; 63) aufweist, der dazu ausgebildet ist, das mit dem Verbindungsaufnahmeteil (67) gekoppelte Verbindungsteil (37) zu einer Bewegung in einer Axialrichtung der Mittelachse (O) anzutreiben und um die Mittelachse (O) zu drehen; und
einen Führungsmechanismus (77), der ein röhrenförmiges Führungselement (82), das härter als der Übertragungsbereich (23d) ist und durch das der Übertragungsbereich (23d) eingeführt werden kann, und ein Halteteil (80) aufweist, das mit der Einführvorrichtung (16) verbunden ist und das röhrenförmige Führungselement (82) hält, wobei das röhrenförmige Führungselement (82) zum Führen und Halten des Übertragungsbereichs (23d) ausgebildet ist, und das Halteteil (80) zum Anordnen des Führungselements (82) in einer Führungsposition ausgebildet ist, in welcher das röhrenförmige Führungselement (82) zwischen dem mit dem Verbindungsaufnahmeteil (67) gekoppelten Verbindungsteil (37) und dem proximalen Endöffnungsbereich (34; 34, 85, 86) in der Axialrichtung der Mittelachse (O) angeordnet ist, und eine Mittelachse des Führungselements (82) in der Führungsposition mit der Mittelachse (O) der Antriebsvorrichtung (54) übereinstimmt,
wobei
das Verbindungsteil (37) am Verbindungsaufnahmeteil (67) angebracht und von ihm entfernt werden kann, und
das Halteteil (80) zum Halten des röhrenförmigen Führungselements (82) so ausgebildet ist, dass das röhrenförmige Führungselement (82) zwischen der Führungsposition und einer Rückzugsposition bewegt werden kann, in der das röhrenförmige Führungselement (82) aus einem Raum zwischen dem mit dem Verbindungsaufnahmeteil (67) gekoppelten Verbindungsteil (37) und dem proximalen Endöffnungsbereich (34; 34, 85, 86) zur Axialrichtung der Mittelachse (O) hin oder zu einer Richtung um eine Drehachse (P) herum parallel zur Längsachse der Einführvorrichtung (16) hin zurückgezogen ist.

2. Medizinisches System nach Anspruch 1, **dadurch gekennzeichnet, dass**
das Führungselement (82) einen Anbringungs- und Entfernungsbereich (84) aufweist, durch den der Übertragungsbereich (23d) am Führungselement (82) in einer Richtung angebracht und von ihm entfernt werden kann, die eine Führungsrichtung kreuzt, entlang welcher der Übertragungsbereich (23d) durch das Führungselement (82) geführt wird.

3. Medizinisches System nach Anspruch 1, **dadurch gekennzeichnet, dass**
das röhrenförmige Führungselement (82) einen Rückzugsausweichbereich (87) aufweist, um den Übertragungsbereich (23d) und/oder den proximalen Endöffnungsbereich (34; 34, 85, 86) so ausweichen zu lassen, dass das röhrenförmige Führungselement (82) den proximalen Endöffnungsbereich (34; 34, 85, 86) und/oder den Übertragungsbereich (23d), der sich zwischen dem mit dem Verbindungsaufnahmeteil (67) gekoppelten Verbindungsteil (37) und dem proximalen Endöffnungsbereich (34; 34, 85, 86) erstreckt, im Hinblick auf die Bewegung des röhrenförmigen Führungselements (82) in der Axialrichtung der Mittelachse (O) zur Rückzugsposition nicht beeinträchtigt.

4. Medizinisches System nach Anspruch 3, **dadurch gekennzeichnet, dass**
der Führungsmechanismus (77) ein Fallschutzelement (89) aufweist, das den Rückzugsausweichbereich (87) von einer Außenseite des Führungselements (82) bedeckt.

5. Medizinisches System nach Anspruch 1, **dadurch gekennzeichnet, dass**
der Halteteil (80) das Führungselement (82) so hält, dass es in der Axialrichtung der Mittelachse (O) beweglich ist,
ein Führungsmechanismus (77) einen Pressmechanismus (89) aufweist, der das Führungselement (82) in der Axialrichtung der Mittelachse (O) zur Seite des Verbindungsaufnahmeteils (67) hin drückt,
das Führungselement (82) einen Kontaktbereich (90b) aufweist, der an einem Endbereich auf der Seite des Verbindungsaufnahmeteils (67) vorgesehen ist, und
das Verbindungsteil (37) einen Kontaktaufnahmebereich (91) aufweist, der am proximalen Endbereich des Übertragungsbereichs (23d) angeordnet ist und mit dem der Kontaktbereich (90b) in Kontakt kommen kann.

6. Medizinisches System nach Anspruch 5, **dadurch gekennzeichnet, dass**
das Führungselement (82) einen Folgeausweichbereich (87) aufweist, um den Übertragungsbereich (23d) und/oder den proximalen Endöffnungsbereich (34; 34, 85, 86) so ausweichen zu lassen, dass das Führungselement (82) den proximalen Endöffnungsbereich (34; 34, 85, 86) und/oder den Übertragungsbereich (23d), der sich zwischen dem mit dem Verbindungsaufnahmeteil (67) gekoppelten Verbindungsteil (37) und dem proximalen Endöffnungsbereich (34; 34, 85, 86) erstreckt, im Hinblick auf die Bewegung des Führungselements (82) in der Axialrichtung der Mittelachse (O) nicht beeinträchtigt.

7. Medizinisches System nach Anspruch 6, **dadurch gekennzeichnet, dass**
der Führungsmechanismus (77) ein Fallschutzelement (89) aufweist, das den Folgeausweichbereich (87) von der Außenseite des Führungselements (82) bedeckt.

8. Medizinisches System nach Anspruch 1, **dadurch gekennzeichnet, dass**
der Halteteil (80) das Führungselement (82) so hält, dass es in der Axialrichtung der Mittelachse (O) beweglich ist,
der Führungsmechanismus (77) einen Pressmechanismus (89), zum Drücken des röhrenförmigen Führungselements (82) in der Axialrichtung der Mittelachse (O) zur Seite des Verbindungsaufnahmeteils (67) hin, und einen Haltemechanismus (97) aufweist, der zum Halten des röhrenförmigen Führungselements (82) in der Rückzugsposition ausgebildet ist,
das röhrenförmige Führungselement (82) einen Kontaktbereich (90b) aufweist, der an einem Endbereich auf der Seite des Verbindungsaufnahmeteils (67) vorgesehen ist, und
das Verbindungsteil (37) einen Kontaktaufnahmebereich (91) aufweist, der am proximalen Endbereich des Übertragungsbereichs (23d) angeordnet ist und mit dem der Kontaktbereich (90b) in Kontakt kommen kann.

9. Medizinisches System nach Anspruch 1, **dadurch gekennzeichnet, dass**
die Behandlungsvorrichtung (35a; 35b) einen Betätigungsmechanismus (30; 36), der im Betätigungsteil (22d, 36; 22d) vorgesehen ist, einen Verbindungsmechanismus (48), der im Verbindungsteil (37) vorgesehen ist, und ein Übertragungselement (47) aufweist, das durch den Übertragungsbereich (23d) eingeführt wird, sich zwischen dem Betätigungsmechanismus (30; 36) und dem Verbindungsmechanismus (48) erstreckt und eine auf den Verbindungsmechanismus (48) aufgebrachte Antriebskraft auf den Betätigungsmechanismus (30; 36) überträgt, um den Betätigungsmechanismus (30; 36) zu betätigen, und
die Antriebsvorrichtung (54) einen Antriebsverbindungsmechanismus (48) zum Antreiben des Verbindungsmechanismus (48) aufweist.

## Revendications

1. Système médical comprenant :
un dispositif d'insertion (16) comportant un axe longitudinal, configuré pour être inséré dans une cavité de corps et incluant un canal (32) s'étendant entre une partie d'extrémité distale du dispositif d'insertion (16) et une partie d'extrémité proximale de celui-ci et une partie d'ouverture d'extrémité proximale (34 ; 34, 85, 86) connectée à une partie d'extrémité proximale du canal (32) ; et
un dispositif de traitement (35a ; 35b) configuré pour être inséré dans le canal (32) depuis la partie d'ouverture d'extrémité proximale (34 ; 34, 85, 86) et pour être inséré à travers le canal (32), et comprenant une partie d'actionnement (22d, 36 ; 22d) prévue au niveau d'une partie d'extrémité distale du dispositif de traitement (35a ; 35b), une partie d'accouplement (37) prévue au niveau d'une partie d'extrémité proximale du dispositif de traitement (35a ; 35b) et prévue à l'extérieur du dispositif d'insertion (16), et une partie de transmission (23d) s'étendant entre la partie d'actionnement (22d, 36 ; 22d) et la partie d'accouplement (37) et configurée pour transmettre une force d'entraînement appliquée à la partie d'accouplement (37) à la partie d'actionnement (22d, 36 ; 22d),
**caractérisé en ce qu'**il comprend en outre :
un dispositif d'entraînement (54) disposé sur le même côté que la partie d'ouverture d'extrémité proximale (34 ; 34, 85, 86) par rapport à l'axe longitudinal du dispositif d'insertion (16) et comportant un axe central (O) parallèle à l'axe longitudinal du dispositif d'insertion (16), le dispositif d'entraînement (54) comprenant en outre une partie de réception d'accouplement (67) configurée pour être couplée avec la partie d'accouplement (37) et un mécanisme d'entraînement (61 ; 63) configuré pour entraîner la partie d'accouplement (37) couplée avec la partie de réception d'accouplement (67) pour se déplacer dans une direction axiale de l'axe central (O) et pour tourner autour de l'axe central (O) ; et
un mécanisme de guidage (77) comprenant un élément de guidage tubulaire (82) qui est plus dur que la partie de transmission (23d) et à travers lequel la partie de transmission (23d) est configurée pour être insérée, et une partie de support (80) qui est connectée au dispositif d'insertion (16) et supporte l'élément de guidage tubulaire (82), l'élément de guidage tubulaire (82) étant configuré pour guider et supporter la partie de transmission (23d), et la partie de support (80) étant configurée pour disposer l'élément de guidage (82) dans une position de guidage, dans laquelle l'élément de guidage tubulaire (82) est disposé entre la partie d'accouplement (37) couplée avec la partie de réception d'accouplement (67) et la partie d'ouverture d'extrémité proximale (34 ; 34, 85, 86) dans la direction axiale de l'axe central (O) et un axe central de l'élément de guidage (82) correspondant avec l'axe central (O) du dispositif d'entraînement (54) au niveau de la position de guidage,
dans lequel
la partie d'accouplement (37) et attachable à et détachable de la partie de réception d'accouplement (67), et
la partie de support (80) est configurée pour supporter l'élément de guidage tubulaire (82) d'une manière telle que l'élément de guidage tubulaire (82) est mobile entre la position de guidage et une position de retrait au niveau de laquelle l'élément de guidage tubulaire (82) est retiré d'un espace entre la partie d'accouplement (37) couplée avec la partie de réception d'accouplement (67) et la partie d'ouverture d'extrémité proximale (34 ; 34, 85, 86) vers la direction axiale de l'axe central (O) ou vers une direction autour d'un axe de rotation (P) parallèle à l'axe longitudinal du dispositif d'insertion (16).

2. Système médical selon la revendication 1, **caractérisé en ce que**
l'élément de guidage (82) comprend une partie d'attache et de détachement (84) à travers laquelle la partie de transmission (23d) est attachée à et détachable de l'élément de guidage (82) dans une direction croisant une direction de guidage le long de laquelle la partie de transmission (23d) est guidée par l'élément de guidage (82).

3. Système médical selon la revendication 1, **caractérisé en ce que**
l'élément de guidage tubulaire (82) comprend une partie d'échappement de retrait (87) pour qu'au moins l'une parmi la partie de transmission (23d) et la partie d'ouverture d'extrémité proximale (34 ; 34, 85, 86) s'échappe d'une manière telle que l'élément de guidage tubulaire (82) n'interfère pas avec au moins l'une parmi la partie d'ouverture d'extrémité proximale (34 ; 34, 85, 86) et la partie de transmission (23d) s'étendant entre la partie d'accouplement (37) couplée avec la partie de réception d'accouplement (67) et la partie d'ouverture d'extrémité proximale (34 ; 34, 85, 86) par rapport au déplacement de l'élément de guidage tubulaire (82) dans la direction axiale de l'axe central (O) vers la position de retrait.

4. Système médical selon la revendication 3, **caractérisé en ce que**
le mécanisme de guidage (77) comprend un élément empêchant la chute (89) couvrant la partie d'échappement de retrait (87) d'un côté extérieur de l'élément de guidage (82).

5. Système médical selon la revendication 1, **caractérisé en ce que**
la partie de support (80) supporte l'élément de guidage (82) de façon à être mobile dans la direction axiale de l'axe central (O)
un mécanisme de guidage (77) comprend un mécanisme de sollicitation (89) sollicitant l'élément de guidage (82) vers un côté d'une partie de réception d'accouplement (67) dans la direction axiale de l'axe central (O),
l'élément de guidage (82) comprend une partie de contact (90b) prévue au niveau d'une partie d'extrémité d'un côté de la partie de réception d'accouplement (67), et
la partie d'accouplement (37) comprend une partie de réception de contact (91) disposée au niveau de la partie d'extrémité proximale de la partie de transmission (23d) et avec laquelle la partie de contact (90b) est configurée pour venir en contact.

6. Système médical selon la revendication 5, **caractérisé en ce que**
l'élément de guidage (82) comprend une partie d'échappement de suivi (87) pour qu'au moins l'une parmi la partie de transmission (23d) et la partie d'ouverture d'extrémité proximale (34 ; 34, 85, 86) s'échappe d'une manière telle que l'élément de guidage (82) n'interfère pas avec au moins l'une parmi la partie d'ouverture d'extrémité proximale (34 ; 34, 85, 86) et la partie de transmission (23d) s'étendant entre la partie d'accouplement (37) couplée avec la partie de réception d'accouplement (67) et la partie d'ouverture d'extrémité proximale (34 ; 34, 85, 86) par rapport au déplacement de l'élément de guidage (82) dans la direction axiale de l'axe central (O).

7. Système médical selon la revendication 6, **caractérisé en ce que**
le mécanisme de guidage (77) comprend un élément empêchant la chute (89) couvrant la partie d'échappement de suivi (87) du côté extérieur de l'élément de guidage (82).

8. Système médical selon la revendication 1, **caractérisé en ce que**
la partie de support (80) supporte l'élément de guidage (82) de façon à être mobile dans la direction axiale de l'axe central (O),
le mécanisme de guidage (77) comprend un mécanisme de sollicitation (89) destiné à solliciter l'élément de guidage tubulaire (82) vers un côté de la partie de réception d'accouplement (67) dans la direction axiale de l'axe central (O), et un mécanisme de maintien (97) configuré pour maintenir l'élément de guidage tubulaire (82) au niveau de la position de retrait,
l'élément de guidage tubulaire (82) comprend une partie de contact (90b) prévue au niveau d'une partie d'extrémité du côté de la partie de réception d'accouplement (67), et
la partie d'accouplement (37) comprend une partie de réception de contact (91) disposée au niveau de la partie d'extrémité proximale de la partie de transmission (23d) et avec laquelle la partie de contact (90b) est configurée pour venir en contact.

9. Système médical selon la revendication 1, **caractérisé en ce que**
le dispositif de traitement (35a ; 35b) comprend un mécanisme d'actionnement (30 ; 36) prévu dans la partie d'actionnement (22d, 36 ; 22d), un mécanisme d'accouplement (48) prévu dans la partie d'accouplement (37), et un élément de transmission (47) inséré à travers la partie de transmission (23d), s'étendant entre le mécanisme d'actionnement (30 ; 36) et le mécanisme d'accouplement (48), et pour transmettre une force d'entraînement appliquée au mécanisme d'accouplement (48) au mécanisme d'actionnement (30 ; 36) pour actionner le mécanisme d'actionnement (30 ; 36), et
le dispositif d'entraînement (54) comprend un mécanisme d'accouplement en entraînement (48) pour entraîner le mécanisme d'accouplement (48).
